# EUROPEAN PATENT APPLICATION

(11) **EP 1 611 903 A1**
(43) Date of publication of application: **04.01.2006**
(21) Application number: 04718407.2
(22) Date of filing: 08.03.2004
(51) Int. Cl.: A61K 49/00

(54) **AGENT FOR DETECTING SENTINEL LYMPH NODE AND DETECTION METHOD**

(30) Priority: 10.03.2003 JP 2003063753
(71) Applicant: Satake, Masanobu, Sendai-shi, Miyagi 989-3204 (JP)
(72) Inventor: TAKEDA, Motohiro, Miyagi 981-3134 (JP); OHUCHI, Noriaki, Miyagi 981-0933 (JP); KAWAZOE, Y., Miyagi 989-3204 (JP); KASUYA, Atsuo, Sendai-shi, Miyagi 981-3122 (JP)
(74) Representative: Kyle, Diana
(86) International application number: PCT/JP2004/002973
(87) International publication number: WO 2004/080491

(57) **Abstract**

The present invention is characterized by easy and rapid detection of a sentinel lymph node by performing sentinel lymph node biopsy with fluorescent particles having a predetermined particle diameter.

## Description

### TECHNICAL FIELD

The present invention relates to an agent for detecting a sentinel lymph node, and a method of detecting a sentinel lymph node using the same.

### BACKGROUND ART

In recent years, due to the progress in early discovery of tumors, surgical treatments of early found malignancies are increasing.

Tumor cells tend to intrude into lymph vessels, metastasize to lymph nodes along with the flow of lymph fluid, and move to other organs. Therefore, even the operations on early found tumors often excise not only the tumors but also the lymph nodes around the tumors, in order to avoid metastasis of the tumor cells. The determination of success of the operation, as well as the preparation of a treatment plan, is made in accordance with the pathological examination of the excised lymph nodes.

In the past, it was difficult to determine how many lymph nodes needed to be excised because there was no means to confirm the metastasis of tumor cells to lymph nodes. Accordingly, many lymph nodes around tumor cells were excised to minimize the risk of tumor metastasis. The excision of many lymph nodes imposed heavy burdens for patients.

Recently, it has been found that metastasis of tumor cells to lymph nodes is little if the tumor is early found carcinoma of the breast or the like. Thus, attempts to minimize excision of lymph nodes has been made by a new type of operation called a sentinel lymph node biopsy. For example, it has been proposed to omit excision of axillary lymph nodes which are considered to be the most easily affected by tumor cells, depending on the results of the sentinel lymph node biopsy, if the tumor is of breast cancer.

The sentinel lymph node biopsy is described below in a detailed manner.

The sentinel lymph node biopsy is a test method to determine whether there is metastasis to a sentinel lymph node that is a lymph node to which a substance intruding into the lymph system of a living body, such as a tumor cell intruded into a lymph vessel from the original tumor of a cancer, first reaches.

A more detailed description is made in the following taking an example in which the substance intruding into the lymph system of a living body is a tumor cell.

It is now known that metastasis of tumor cells to lymph nodes does not happen randomly but shows a certain pattern. Specifically, after intrusion of tumor cells into a lymph vessel from the original tumor, metastasis happens in a sentinel lymph node which is the closest lymph node from the intrusion point of the lymph vessel. Further, subsequent metastasis happens at another lymph node which is the closest to the sentinel lymph node. Then, this type of metastasis repeats. Thus, it is believed that the sentinel lymph node necessarily has metastasis if tumor cells spread into lymph nodes. For confirmation, it is noted that "sentinel" means a guard, sentry, or the like.

Accordingly, it is possible to determine the presence or absence of metastasis to lymph nodes of tumor cells by, for example, performing a sentinel lymph node biopsy in which a sentinel lymph node is found, excised, and subjected to a pathological test. If no metastasis of tumor cells is found in the sentinel lymph node, additional excision of lymph nodes can be avoided. For instance, in the case of breast cancer, dissection of axillary lymph nodes can be omitted. On the other hand, if metastasis of tumor cells in the sentinel lymph node is present, a tissue including the sentinel lymph node or a tissue including a plurality of more lymph nodes located at a lower side of the sentinel lymph node, depending on the extent of the metastasis, is cut off.

As will be understood from the above, a patient who has no metastasis of tumor cells to lymph nodes can avoid unnecessary dissection of lymph nodes by having a sentinel lymph node biopsy performed. Therefore, physical burdens during operations are reduced. This method is applicable not only to breast cancer but also to other surgical procedures with lymph node dissection.

Since a sentinel lymph node biopsy is performed during an operation such as a tumor extraction operation, due to limited time of the operation, a sentinel lymph node needs to be identified easily and rapidly when performing the sentinel lymph node biopsy. At present, generally speaking, two types of methods are used to identify sentinel lymph nodes.

The first method is called a dye method which topically injects a dye such as isosulphan blue around a tumor during the operation. The injection can be made by use of percutaneous injections, endscopes or the like. The injected dye intrudes into a lymph vessel from the injected point, and reaches a sentinel lymph node within some minutes or ten plus some minutes. The sentinel lymph node can be identified by visually identifying a lymph node which was first colored.

However, lymph nodes are often covered by living tissue such as fat. Therefore, a search for a sentinel lymph node often needs additional operations of division of the surrounding tissue. If the search takes time, the dye may reach lymph nodes down from the sentinel lymph node during the search time, which would make the identification of the sentinel lymph node difficult. Further, the dye method cannot identify a sentinel lymph node from the outside of the body. Furthermore, some dyes may cause allergic reactions in a patient.

The second method is to topically inject a small amount of radioisotope (RI) having a short lifetime such as 99m-technetium around a tumor before starting an operation. This is called an isotope method. Like in the first method, the injection can be made by use of percutaneous injections, endscopes, or the like. The injected radioisotope intrudes into a lymph vessel from the injected point, and remains at a sentinel lymph node for a certain amount of time. Therefore, by detecting gamma-rays using a gamma-probe during an operation, a lymph node which irradiates the most intensive gamma-rays can be identified as the sentinel lymph node.

In this method, the radioisotope does not pass through the sentinel lymph node during a short period of time. And, it is possible to detect a sentinel lymph node covered by other tissue. Therefore, accuracy of the sentinel lymph node detection is high. However, it requires a complicated operation system because expensive radioisotopes and a gamma-probe are used. Accordingly, it is difficult to perform the radioisotope method in a hospital other than a large-scale hospital which is capable of handling radioisotopes. In addition, it takes time because the detection using a gamma-probe cannot visualize emission of radiation which results in blind detection of lymph nodes.

### DISCLOSURE OF THE INVENTION

An object of the present invention is to solve the problems in the prior art described above.

Specifically, an object of the present invention is to provide a detection agent which can identify a sentinel lymph node from the outside of the body, and can more easily and more rapidly identify a sentinel lymph node as compared to the above-described dye method and isotope method.

In addition, another object of the present invention is to provide a method of detecting a sentinel lymph node using the detection agent.

The objects of the present invention can be achieved by performing a sentinel lymph node biopsy by use of fluorescent particles having a determined diameter.

For example, for human beings, fluorescent particles having a diameter from equal to or more than 200 nm to equal to or less than 1000 nm are used as an agent for detecting a sentinel lymph node. For small mammals other than human beings, fluorescent particles having a diameter from equal to or more than 40 nm to less than 200 nm are used as an agent for detecting a sentinel lymph node.

It is preferable that the fluorescent particles be those which emit fluorescence having a wavelength from 600 to 900 nm after irradiation by excitation energy. At least one part or an entirety of the surface of the fluorescent particles may be constituted by organopolysiloxane.

Specifically, the sentinel lymph node detecting method according to the present invention is characterized by comprising:
a step of injecting into a living body fluorescent particles;
a step of radiating excitation energy near the injected point, in particular in the area at which lymph nodes are present; and
a step of detecting fluorescence emitted from the fluorescent particles.

It is preferable to use fluorescent particles having a diameter equal to or more than 40 nm and less than 200 nm, and those having a diameter equal to or more than 200 nm and equal to or less than 1000 nm, for small mammals other than human beings and for human beings, respectively.

### BRIEF DESCRIPTION OF THE DRAWINGS

Fig. 1 is a CCD camera image obtained in Example 1 to be explained later.
Fig. 2 is a CCD camera image obtained in Comparative Example 1 to be explained later.

### BEST MODE FOR CARRYING OUT THE INVENTION

The sentinel lymph node biopsy according to the present invention has as a main feature that particles having a predetermined diameter range and being capable of emitting fluorescence after excitation are introduced into lymph fluid in a living body of a human being or small mammal other than a human being, and a lymph node which the particles first reach is identified as a sentinel lymph node. According to the present invention, "small mammals" mean mammals having a size equal to or less than human beings, such as rats, dogs, cats, monkeys, pigs, goats, rabbits, sheep, and the like.

The lymph system in the body of human beings and small mammals other than human beings has lymph vessels and nodes having material conveyance functions and filter functions which are intrinsic to animal species. In the case of human beings, materials having a diameter of about 200 nm or more cannot pass through a lymph node during a short period of time. In the case of small mammals other than human beings, the same applies to materials having a diameter of about 40 nm or more. Accordingly, big particles which have reached a lymph node by a lymph fluid stream in a living body but cannot pass it are supposed to remain in the lymph node.

Therefore, for example, in the case of a human being, a sentinel lymph node can be identified if particles having a diameter of 200 nm or more are injected around a tumor in the body of human being, and are traced along with the movement thereof by the lymph fluid stream to detect a lymph node in which the particles remain. In the case of small mammals other than human beings, detection of a sentinel lymph node can be performed as above by reducing the diameter of the particles to 40 nm or more. If particles having a size less than 200 nm (for a human being) or 40 nm (for a small mammal other than a human being) are used, they do not remain in the lymph nodes in the living body, and pass onward in a short time. Therefore, the detection of a sentinel lymph node cannot be made sufficiently during the time of an operation (however, even so, visualization of lymph vessels is possible).

On the other hand, if the diameter of particles to be introduced into the lymph system of the living body is too large, the movement of the particles in the lymph vessels may take time, so that the detection of a sentinel lymph node during the operation time may be difficult. Accordingly, the diameter of the particles as an agent for detecting a sentinel lymph node is inevitably limited. The upper limit of the diameter is about 1000 nm for human beings, and is about 200 nm for small mammals other than human beings.

Consequently, the diameter of the agent particles for detecting a sentinel lymph node according to the present invention is equal to or more than about 40 nm and less than 200 nm for small mammals other than human beings, and is equal to or more than about 200 nm and equal to or less than about 1000 nm for human beings. For human beings, the diameter thereof is preferably equal to or more than 300 nm and equal to or less than 800 nm, more preferably equal to or more than 400 nm and equal to or less than 700 nm, and even more preferably equal to or more than 500 nm and equal to or less than 600 nm. For small mammals other than human beings, the diameter thereof is preferably equal to or more than 40 nm and equal to or less than 150 nm, more preferably equal to or more than 40 nm and equal to or less than 100 nm, and even more preferably equal to or more than 40 nm and equal to or less than 80 nm.

The term "particle" according to the present invention also includes imperfect spheres such as ellipsoids as well as perfect globes. For the imperfect spheres, "diameter" means the length of a particle along with the longest size dimension of the particle.

The particles for the detection agent of a sentinel lymph node according to the present invention may have the same particle diameter, or may have a particle size distribution within the particle range described above. Since the time necessary for the particles to reach a sentinel lymph node depends on the diameter of the particles, it is preferable for the particles that they all have approximately the same diameter in view of the control of the arrival time.

The particles to be used as the agent for detecting a sentinel lymph node according to the present invention are fluorescent particles having a nature of emitting fluorescence after irradiation by excitation energy. There is no limitation on the kinds of the excitation energy, and heat, a magnetic field, light or the like can be listed. Light is preferable, and irradiation by laser light having a wavelength shorter than that of the fluorescence is in particular preferable. As the laser light, the light from laser oscillators such as diode lasers, He-Ne lasers, and the like, can be used.

It is preferable that the wavelength of the fluorescence emitted from the particles as the agent for detecting a sentinel lymph node according to the present invention be in a range from 600 to 900 nm. If it is less than 600 nm, high sensitivity measurement becomes difficult because it overlaps with the wavelength of the fluorescence emitted from fluorescent materials, such as porphyrin, which are naturally present in a living body (autonomous fluorescence). If the fluorescence wavelength is more than 900 nm, the kinds of available fluorescent particles are limited, and this is not preferable in view of practicality. The fluorescence wavelength is preferably from 620 to 800 nm, more preferably 650 to 700 nm, and even more preferably 660 to 680 nm.

Thus, if the fluorescence wavelength of the fluorescent particles for detecting a sentinel lymph node is in a range from 600 to 900 nm, the wavelength of the excitation energy light is preferably less than 600 nm. Likewise, if the fluorescence wavelength is in a range from 620 to 800 nm, 650 to 700 nm, and 660 to 680 nm, the wavelength of the excitation energy light is preferably less than 620 nm, less than 650 nm, and less than 660 nm, respectively. Generally, an absorbent peak of the excitation energy light is present at the wavelength which is shorter by 20 to 30 nm than the wavelength at the peak of fluorescence. However, in order to facilitate the detection of the fluorescence avoiding overlap between the fluorescence to be detected and the excitation energy light, excitation of the fluorescent substance at a further shorter wavelength is preferable.

The fluorescent substance in the fluorescent particles can be made from any material of inorganic or organic type as long as it comprises a substance which is capable of emitting fluorescence preferably with the above wavelength.

As the inorganic fluorescent substances, for example, florescent substances made from metal elements in Group III to XVI, in particular compounds of rare earth metals, can be mentioned. In terms of the intensity of fluorescence, compounds of trivalent europium, trivalent terbium, trivalent samarium, trivalent dysprosium and divalent europium are preferable. These may be used alone or in combination of two or more. The inorganic fluorescent substances can be excited at a wavelength much shorter than that of their fluorescence, and therefore overlap of excitation light and fluorescence is small. Thus, the inorganic fluorescent substances are preferable because of their properties regarding wavelength for excitation.

As the organic fluorescent substances, for example, various dyes such fluorescein, acriflavin, rhodamines, 3,3-diethyltricarbocyanine iodide, chroroaluminum phthalocyanine, 5-carboxyfluoroscein, 5-(2'-aminoethyl)aminonaphthalene-1-sulfonic acid, anthranylamides, coumalins, cyanine dyes, BODIPY dyes as can be mentioned. Specifically, acidic fuchsine, Acridine Orange, Acridine Red, Acridine Yellow, acriflavin, Alizarin Red, allophycocyanine, Aminoactinomycin D, 7-Aminoactinomycin D-AAD, Astrazon Brilliant Red 4G, Astrazon Red 6B, Astrazon Orange, aminocoumalin, anthryl stearate, atabrine, auramine, Aurophosphine, Aurophosphine G, berberine sulfate, bisbenzamide, CY 3.18, CY 5.18, CY 7, Erythrosine ITC, ethydium bromide, Frazo Orange, FM 1-43, Genacryl Brilliant Red B, Genacryl Pink 3G, Lisamine Rhodamine B200, Lysotracker Yellow, Lysotracker Red, Magdala Red, Magnesium Orange, mythramycin, Nile Red, nitrobenzoxadiazole, parasosanillin, Phosphine 3R, Phosphine R, Pontchrome Blue Black, Primlin, Procion Yellow, propidium iodide, Pyronine B, R-Phycoerythin, Rhodamine 5 GLD, Rhodamine 6G, Rhodamine B, Rhodamine B200, Rhodamine B Extra, Rose Bengal, serotonin, Sevron, Brilliant Red 2B, Sulphorhodamine G Extra, Thiazine Red R, Thioflavin S, Ultralight, Xyleneorange, and the like can be cited.

As the matrix for bearing the fluorescent substances in the fluorescent particles, any material of inorganic or organic type can be used. As inorganic materials, silica, talc, calcium carbonate, magnesium carbonate, aluminum oxide, zinc oxide, barium sulfate, glass, fullerenes such as carbon fullerene, nanotubes such as carbon nanotube, zeolite, and the like can be cited. As organic materials, radical polymerization type polymers such as polyethylene, polypropylene, polystyrene, polymethylmethacrylate, polyacrylamide, polyvinyl chloride, polyvinylidene chloride; polycondensation type polymers such as polyester, polyamide, polyimide, polycarbonate; polyaddition type polymers such as polyurethane; organopolysiloxanes such as silicone rubber; and the like can be cited. Among these, in terms of availability, organic materials are preferable. Furthermore, in terms of biostability and biosafety, radical polymerization type polymers such as polystyrene and organopolysiloxanes are preferable. The matrix may be made from various biodegradable organic polymers such as polylactic acid if the fluorescent particles which may remain in a living body after the detection of a sentinel lymph node are to be early degraded and disposed of.

As commercially available products to be used as the fluorescent particles, for example, FluoroSphere ® that is an aqueous suspension of 2% polystyrene matrix type fluorescent beads (fluorescent substance: various dyes) sold by Molecular Probes, may be cited.

The surface of the fluorescent particles as the agent for detecting a sentinel lymph node according to the present invention may be subjected to various treatments such as those for making it hydrophilic or hydrophobic, and those for introducing functionality. As treatment for hydrophilicity, plasma treatment, UV treatment or the like can be cited. As treatment for hydrophobicity, surface treatments by organopolysiloxanes such as methylhydrogenpolysiloxane, perfluoroalkylphosphates or the like can be cited. As functional groups to be introduced onto the surface of the particles, carboxylate groups, sulfate groups, aldehyde sulfate groups, amino groups, and the like, can be cited, but there is no limitation to these.

It is preferable for the fluorescent particles used by the present invention that at least a part of the surface thereof be constituted by organopolysiloxanes in view of bioadaptability and biostability. Specifically, the matrix of the fluorescent particles may be organopolysiloxanes such as silicone rubber. Alternatively, at least a part of the fluorescent particles may be surface-treated with organopolysiloxanes. The entire surface of the fluorescent particles may be covered with organopolysiloxanes.

The method of detecting a sentinel lymph node according to the present invention can be performed by selecting the diameter of fluorescent particles from the above different numerical ranges depending on which of human beings or small mammals other than human beings the subject is, injecting the fluorescent particles having a predetermined diameter into the living body, then radiating excitation energy near the injected point, and detecting fluorescence emitted from the fluorescent particles.

Since the radiation of the fluorescent particles is intense, the fluorescence can be visually observed through the skin with the naked eye. Therefore, by simply checking the area from which fluorescence emits, identification of a sentinel lymph node can be easily and rapidly made. Of course, tracing the emission from the fluorescent particles with the skin incision is possible. In the latter case, more intense fluorescence can be directly checked, and therefore the identification of a sentinel lymph node becomes easier.

The larger the diameter of the fluorescent particles injected into a living body is, the later the arrival time of the particles to a sentinel lymph node is. Therefore, if the time for an operation is long, it is possible to use fluorescent particles having a relatively large diameter. On the other hand, if identification of a sentinel lymph node within a short time is necessary, those having a relatively small diameter can be used.

Before injecting the fluorescent particles into a living body, lymphoscintigraphy may be performed in order to limit the area to which excitation energy is radiated or that to be searched for a sentinel lymph node. By performing lymphoscintigraphy, it is possible to predict the area in which a sentinel lymph node may be present. The limitation of excitation energy radiation to the predicted area may lower the amount of energy to be used, and the sentinel lymph node biopsy may become more rapid and certain.

The operation, for example tumor extraction operation, with the use of the agent for detecting a sentinel lymph node according to the present invention may be performed as follows.

A suspension of the above fluorescent particles with a predetermined concentration is injected around the tumor or hypodermis on the tumor of a patient. It is not preferable to inject into the original tumor because the injection may cause the spread of tumor cells to the surrounding organs. As the means for the injection, a syringe may be preferably used. If necessary, lymphoscintigraphy may be performed around the tumor before the injection of the fluorescent particles.

After the passage of an appropriate time, for example 10 to 30 minutes, the sentinel lymph node biopsy is started. First, the fluorescent agent is injected around the tumor of the patient. The skin near the tumor or the area in which a sentinel lymph node is predicted to be present is cut, and excitation energy such as laser is radiated to the living tissue around the tumor. Simultaneously, fluorescent parts are scanned. If a tumor is present at the position relatively close to the hypodermis as in the case of skin or breast cancer and the like, the fluorescent parts may be scanned after radiating excitation energy such as a laser onto the skin near the tumor before cutting the skin.

Next, the sentinel lymph node which is identified by the fluorescence is excised and subjected to a tissue test immediately. Then, a lymph node dissection is performed. If metastasis of tumor cells is determined by the tissue test to be negative, only the tumor and surrounding tissue are removed, and the operation ends after the suture of the cut area. On the other hand, if metastasis of tumor cells is determined by the tissue test to be positive, the tumor and a part or entirety of lymph nodes around the tumor are excised. Then, the cut area is closed to end the operation.

### INDUSTRIAL APPLICABILITY

Being different from radioisotopes, the agent for detecting a sentinel lymph node requires no large scale equipment for sentinel lymph node biopsy because the agent does not have radioactivity so that the use thereof is easy. Therefore, tumor excision operations with sentinel lymph node biopsy become possible even in small hospitals. Further, by use of the sentinel lymph node biopsy, burdens of a patient during the excision operation can be reduced because the unnecessary excision of living tissue such as lymph nodes can be avoided. Furthermore, this is not the case with the isotope method, but the present invention can visualize the sentinel lymph node with fluorescence, so that a doctor who does not have very long experience can easily identify the sentinel lymph node.

The fluorescent particles used as the agent for detecting a sentinel lymph node according to the present invention can easily move in a lymph vessel, and have a predetermined diameter which prevents the passage thereof through a lymph node to which they firstly reach. Therefore, they reliably remain in a sentinel lymph node. Accordingly, the sentinel lymph node can be identified with certainty with a simple operation of visually identifying a lymph node emitting fluorescence.

And, the present invention can utilize more time for identifying a sentinel lymph node as compared to the dye method which requires quick identification of a sentinel lymph node. Therefore, the identification by the present invention is easy and reliable. Furthermore, the identification process may be remarkably simplified as compared to that in the dye method because the present invention can realize identification of a sentinel lymph node from the outside of the body.

### Examples

In the following, the present invention is described in detail by way of Examples. It should be understood that the present invention is not limited to these Examples.

### Example 1

A rat (Donryu; female, age of 7 to 10 weeks) was put under anesthesia with aspiration of 6 ml of vaporized diethylether permeated into cotton wool. After the completion of the anesthesia, the lower limbs were shaved.

0.05 ml of an undiluted liquid of Fluosphere Dark Red sold by Molecular Probe including fluorescent particles with unique particle diameter of 40 nm was percutaneously injected at the back of one of the lower limbs. Then, the skin of the lower limb was peeled.

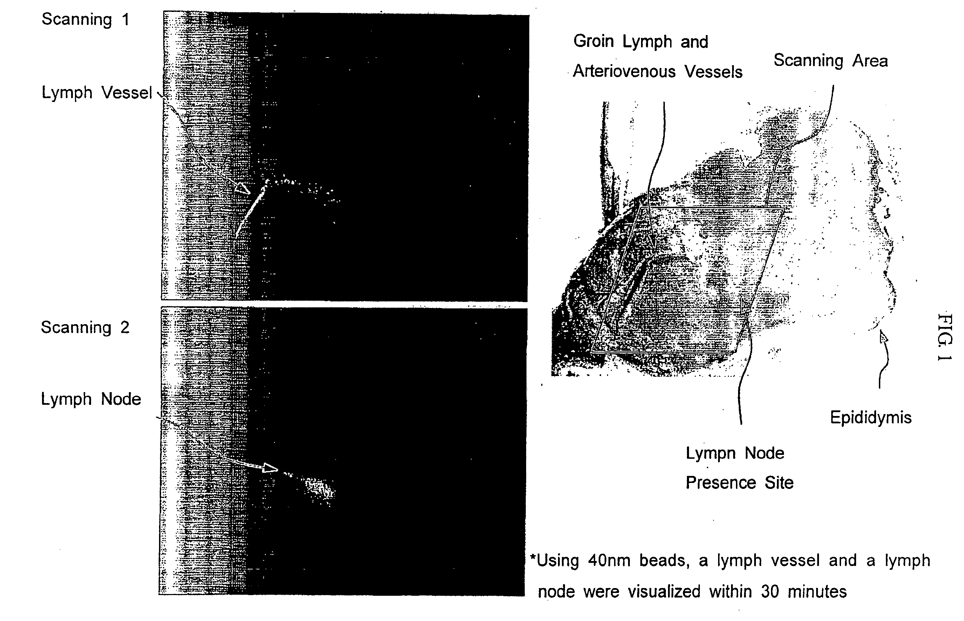

Laser light having a wavelength of 633 nm (obtained by use of He-Ne type device sold by Spectra Physics) was radiated onto the lower limb, and the image of the root of the lower limb was captured by a CCD camera with a band-pass filter for 670 ± 10 nm sold by SONY. A combination of the laser light radiation and the image capture was repeated at 17 minutes and 28 minutes after percutaneous injection. The results are shown in Figure 1. In Figure 1(Scanning 1), which was an image 17 minutes after the percutaneous injection, a lymph vessel in which the fluorescent particles were moving was visualized. In Figure 1(Scanning 2), which was an image 28 minutes after the percutaneous injection, a sentinel lymph node in which the fluorescent particles aggregated was visualized.

### Comparative Example 1

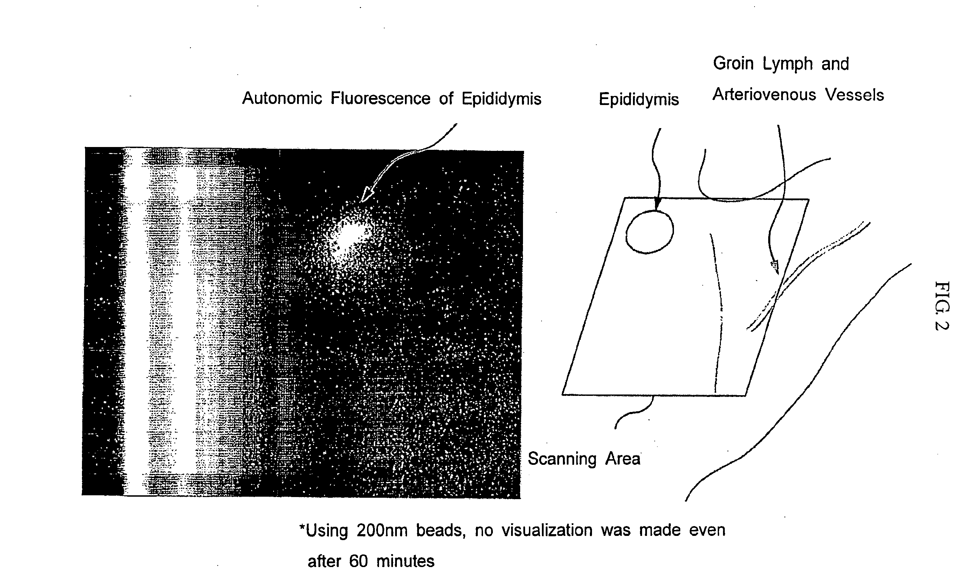

The same processes as in Example 1 were repeated except that the diameter of the fluorescent particles was set to be 200 nm. The results are shown in Figure 2. As obvious from Figure 2, a sentinel lymph node was not visualized within 1 hour after the injection. The fluorescence at the upper right of Figure 2 was luminescence by autonomic fluorescence of the epididymis. A combination of the laser light radiation and the image capture was repeated again 60 minutes after the percutaneous injection. However, a sentinel lymph node was not visualized.

As clear from Figures 1 and 2, when using fluorescent particles having a diameter of 40 nm, a sentinel lymph node was able to be identified within 30 minutes, however, when using fluorescent particles having a diameter of 200 nm, identification of a sentinel lymph node was not possible within 1 hour.

## Claims

1. An agent for detecting a sentinel lymph node of a human being, comprising fluorescent particles having a diameter of equal to or more than 200 nm and equal to or less than 1000 nm.

2. An agent for detecting a sentinel lymph node of a small mammal other than a human being, comprising fluorescent particles having a diameter of equal to or more than 40 nm and less than 200 nm.

3. The agent for detecting a sentinel lymph node according to Claim 1 or 2, **characterized in that** the fluorescent particles emit fluorescence having a wavelength from 600 to 900 nm.

4. The agent for detecting a sentinel lymph node according to any one of Claims 1 to 3, **characterized in that** at least a part of the surface of the fluorescent particles is constituted by organopolysiloxane.

5. A method for detecting a sentinel lymph node of a small mammal other than a human being, **characterized by** comprising:
a step of injecting into a living body of the small mammal other than a human being fluorescent particles having a diameter of equal to or more than 40 nm and less than 200 nm;
a step of radiating excitation energy near a point of the injection on the small mammal; and
a step of detecting fluorescence emitted from the fluorescent particles.
